# EUROPEAN PATENT SPECIFICATION

(11) **EP 2 142 106 B1**
(45) Date of publication and mention of the grant of the patent: **21.09.2016**
(21) Application number: 08733445.4
(22) Date of filing: 02.05.2008
(51) Int. Cl.: A61B 17/072, A61B 17/064, A61B 17/115, A61B 17/11, A61B 17/068, A61B 17/00

(54) **APPARATUS FOR DELIVERING SHAPE MEMORY ALLOY STAPLES**
GERÄT ZUR ABGABE VON KLAMMERN AUS EINER FORMGEDÄCHTNIS-LEGIERUNG
APPAREIL POUR DISTRIBUER DES AGRAFES EN ALLIAGE À MÉMOIRE DE FORME

(30) Priority: 02.05.2007 AU 2007902314 P
(43) Date of publication of application: 13.01.2010
(73) Proprietor: Endogene Limited, Brighton, VIC 3186 (AU)
(72) Inventor: Soutorine, Mikhail, Oakleigh, VIC 3166 (AU); Chernov-Haraev, Artem N., Moscow 127083 (RU)
(74) Representative: Vinsome, Rex Martin
(86) International application number: PCT/AU2008/000623
(87) International publication number: WO 2008/134812

(56) References cited:
- EP-B1- 0 326 757
- WO-A1-00/40159
- US-A- 4 485 816
- US-A- 4 550 870
- US-A1- 2005 070 924
- US-A1- 2005 070 924
- US-B2- 7 004 958
- US-B2- 7 004 958

## Description

### Related Applications

This application claims priority from Australian Patent Application No. 2007902314, filed 2 May 2007.

### Field of the invention

The invention relates generally to an apparatus for fastening particularly, but not exclusively, for suturing in medical applications.

### Background of the invention

It is known to join tissue in medical applications using sutures which may be in the form of a series of threaded stitches. The stitches usually require a surgeon to have reasonably free access to a suture site in order to allow the surgeon to manipulate a threading needle. Manual sewing of the stitches can be somewhat difficult and time consuming in some circumstances. An alternative form of suturing uses staples which are driven out of a head of a stapler device, through the tissue, and then anchored in place by being deformed on an opposed anvil of the stapler device.

Another alternative form of suturing utilises a stapler that includes a curved shaping section that serves to deform the staples into a looped configuration as the staples exit the stapler. A disadvantage of such a stapler is that it is difficult to make the stapler tip of small diameter since the shaping section presents a significant radial dimension to the stapler. A large diameter tip can make many vascular procedures (coronary artery bypass graft, etc.) practically impossible with such a stapler. Another disadvantage associated with the stapler is the discharged staples are not formed in particularly tight loops in comparison to a hand made suture, where the surgeon can tighten the knot after penetrating the tissues with a needle. A further disadvantage associated with the stapler is its inability to form end-to-end and end-to-side joints of tubular bodies with multiple joining staples.

US 2005/0070924 A1 discloses an apparatus according to the preamble of claim 1.

### Summary

According to the present invention, there is provided an apparatus comprising:
a plurality of chambers for receiving an associated plurality of staples, each formed with a shape memory that allows the staple to adopt a straightened configuration, when placed in a stapler, and a deployed configuration for suturing when released from the stapler, characterized in that the apparatus further comprises:
a sleeve moveable relative to the chambers between a first position in which the staples are trapped by the sleeve within the chambers, in the straightened configuration, and a second position whereby the staples are freed to adopt the deployed configuration, wherein the sleeve is adapted to move between the first and second positions by rotating relative to the chambers.

The shape memory may be at least partially thermally activated.

The apparatus may be in the form of a medical stapler.

The apparatus may include a cap for holding a graft on an end of the stapler until the staples are deployed.

### Brief Description of the Drawings

The invention is described, by way of non-limiting example only, with reference to the accompanying drawings, in which:
Figure 1 is a cross-sectional view of a fastening apparatus;
Figure 2a is a perspective partial cross-section of the dispensing tip of the apparatus;
Figure 2b is a perspective partial cross-section of the dispensing tip showing partially discharged staples; and
Figures 2C is a perspective partial cross-section of the dispensing tip showing the staples fully discharged.

### Detailed Description

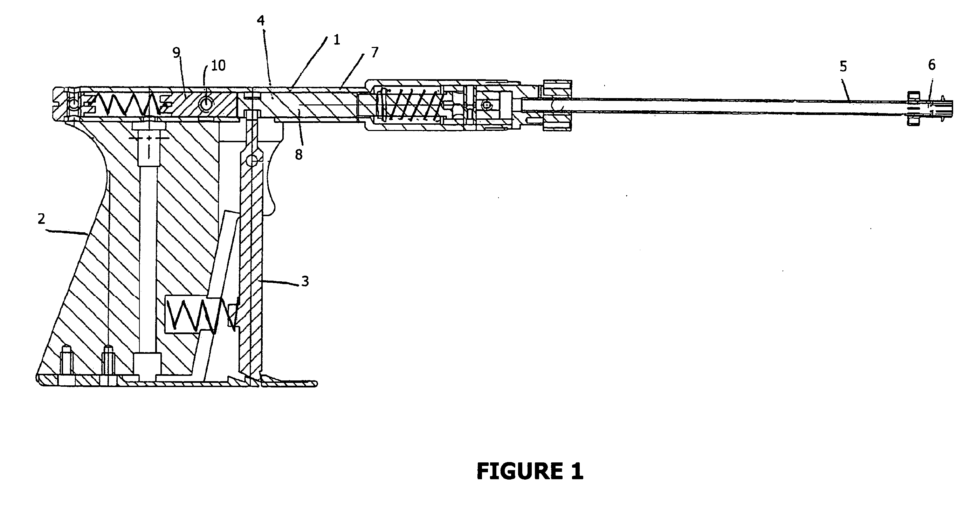

A fastening apparatus 1, which is in the form of a stapler, is shown in Figure 1 as including a hand piece 2 with a trigger 3 coupled to a drive mechanism 4 which is in turn connected to an internal staple carrier located within an elongate sleeve 5 that terminates at a dispensing tip 6.

The drive mechanism 4 is housed in a barrel 7 of the hand piece 2 and includes a shuttle piece 8 which is coupled to a trigger 3 and travels lengthwise of the barrel 7, in response to the positioning of the trigger 3. The shuttle piece 8 is connected to a rotary member 9 which has a lateral lever 10 that extends externally of the barrel 7. The lever 10 allows the rotary member 9 and thereby the entire drive mechanism 4 to be rotated, such as by hand actuation, after the trigger 3 has initially been squeezed, so as to rotate the carrier relative to the sleeve at the dispensing tip 6. Accordingly, when the trigger 3 is activated, the drive mechanism 4 moves to the right, as shown, and is then rotated using lever 10.

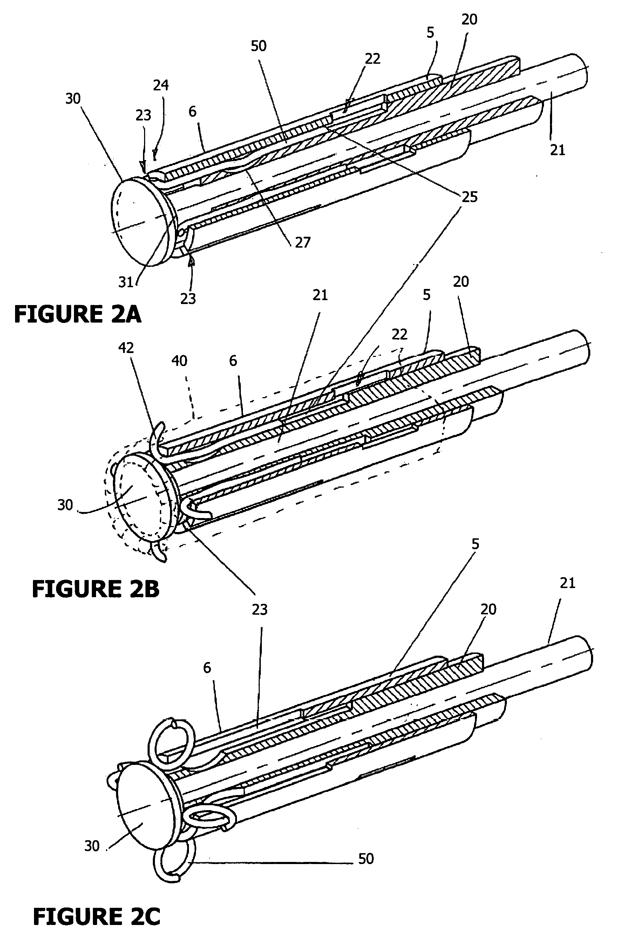

Referring now to Figure 2, the dispensing tip 6 of the apparatus 1 is described in more detail. The tip 6 is formed of a co-axial arrangement of the sleeve 5, staple carrier 20 and a central rod 21, which are all coupled back to the hand piece 2.

The sleeve has a plurality of axially extending elongate openings 22 which serve as entry points for staples to be loaded into the carrier 20. Elongate exit grooves 23 communicate with an open end 24 of the sleeve 5. The grooves 23 are radially offset from the openings 22.

The carrier 20 has a plurality of chambers 25 which are arranged to initially align with the openings 22, for loading the staples into the carrier and to subsequently align with the grooves 23 when the sleeve is rotated relative to the carrier 20 for discharge of the staples. Each chamber 25 includes a small detent 27 into which the associated staple is free to deform, as a means of restricting the staple from premature discharge.
The rod 21 is adapted for limited relative axial movement through the sleeve 6 and is fitted with a cap 30 which has an outwardly flared neck 31 to initially deform the staple, if necessary, and encourage the staple into a curved configuration as the staple is deployed.
In use of the apparatus 1, staples are firstly inserted into the carrier 20 so that each chamber 25 is loaded with a staple. A graft 40, such as a stent or the like, is then fitted over the tip 6 so as to be carried by the cap 30. The trigger 3 is then activated to move the carrier 20 slightly forward to allow the leading ends 42 of the staples 50 to project out from underneath the sleeve 5 and start curling backward. At the same time, the staples puncture the graft 40 and thereby serve to anchor the graft 40 to the tip 6.

The apparatus is then manipulated to place the graft 40 at an appropriate suturing site, at which time the trigger 3 is depressed further so that the grooves 23 align with the chambers 25 to allow the staples to discharge from the tip 6. The apparatus is then removed.
The staples are preferably formed of suitable memory-shape material, such as Nitinol or any other memory-shape material, and preferably also are at least partially thermally active to further constrict and effect a tight suture, once the staples are warmed to body temperature. In order for the staple to form the circular shape -the memory-shape material should be pre-manufactured ("trained") in form of a ring of different diameter depending on the thickness of the joining tissues.

The length of the staple can be different in relation to the diameter of the resultant ring formed thereby, with at least 25% overlap for a more secure connection. The diameter of the staple wire itself (Nitinol wire, etc.) can be of different diameter as well in regards to the thickness and density of connecting tissues, when different penetration force is required.

The above dispensing mechanism has been described with reference to the carrier being moved relative to the sleeve, however, the stapler could instead include a pushing rod for advancing the staples, arranged in the straightened configuration within the chambers, out of the stapler allowing them to obtain the deployed (looped) configuration.
Alternatively, the hand piece can be a kind of lever or a piston or combination of both to which the force of a hand is applied in order to produce the pulling or rotation force along the external sleeve. Further, the tip and hand piece can be joined by a rigid or flexible shaft which allows insertion of the dispenser into the arterials and other areas of body.
In relation to the present embodiment, the dispensing operation need not necessarily be done in the two step process described. Dispensing may instead be done by either a single step of moving the external sleeve 5 and carrier 20 lengthwise of each other; or relatively rotating the external sleeve 5 around the carrier 20 to bring the chambers 25 into alignment with the grooves 23 to allow the staples to be discharged.
In another embodiment (not shown) the cap 30 can be pulled inside of the carrier 20, with the help of rod 21 to increase the diameter of the dispensing tip 1 for a better adaption of the stapler tip (dispenser) toward surrounding body tissues. The cap 30 can also be provided with a cutter which makes a hole (punch) in the tubular organ (vessel) where suturing is required.

The configuration of the dispensing tip 6 and the positioning of the chambers 25 can be varied, as required, to allow the tip 6 to be inserted into tubular bodies where suturing is required, allowing formation of "end to end" or "end to side" joints of the tubular bodies with multiple joining staples. In this case all the staples can be formed simultaneously.

Apart from surgical applications the apparatus of this invention can be used for joining any types of soft materials such as fabrics, leather, cardboard, paper, plastics etc. in other than medical applications, for example, in relation to toys, apparel and all sorts of piping.

## Claims

1. An apparatus comprising:
a plurality of chambers (25) for receiving an associated plurality of staples (50), each formed with a shape memory that allows the staple to adopt a straightened configuration, when placed in a stapler, and a deployed configuration for suturing when released from the stapler,
**characterized in that** the apparatus further comprises:
a sleeve (5) moveable relative to the chambers between a first position, in which the staples are trapped by the sleeve within the chambers in the straightened configuration, and a second position, whereby the staples are freed to adopt the deployed configuration, wherein the sleeve is adapted to move between the first and second positions by rotating relative to the chambers.

2. The apparatus as claimed in claim 1, wherein the shape memory is at least partially thermally activated.

3. The apparatus of claim 1 or claim 2, wherein the sleeve has a plurality of axially extending elongate openings (22) which serve as entry points for staples to be loaded into the chambers and wherein the sleeve has a plurality of elongate exit grooves (23) for discharge of the staples into the deployed configuration.

4. The apparatus of claim 3, wherein the exit grooves are radially offset from the openings.

5. The apparatus of any one of claims 1 to 4, further comprising a carrier (20) that comprises the chambers and the chambers are arranged to initially align with the openings for loading the staples into the carrier and to subsequently align with the exit grooves when the sleeve is rotated relative to the carrier for discharge of the staples.

6. The apparatus of any one of claims 1 to 5, further comprising means for restricting the staples from premature discharge.

7. The apparatus of any one of claims 1 to 5, wherein each chamber (27) defines a detent (26) into which the associated staple is free to deform.

8. The apparatus of any one of claims 1 to 7, further comprising a lever, a drive mechanism (4) and hand piece (2) with a trigger (3), wherein the lever allows the drive mechanism (4) to be rotated after the trigger (3) has been initially activated.

9. An apparatus as claimed in claim 1, in the form of a medical stapler.

10. The apparatus as claimed in claim 9 including a cap (30) for holding a graft (40) on an end of the stapler until the staples are deployed.

## Patentansprüche

1. Gerät, umfassend:
eine Mehrzahl von Kammern (25) zur Aufnahme einer zugehörigen Mehrzahl von Klammern (50), die jeweils mit einem Formgedächtnis ausgebildet sind, das es der Klammer ermöglicht, bei Anordnung in einem Klammerapparat eine gestreckte Konfiguration und bei Freigabe aus dem Klammerapparat eine Einsatzkonfiguration zum Nähen anzunehmen,
**dadurch gekennzeichnet, dass** das Gerät ferner umfasst:
eine Hülse (5), die in Bezug auf die Kammern zwischen einer ersten Position, in welcher die Klammern durch die Hülse innerhalb der Kammern in der gestreckten Konfiguration eingeklemmt sind, und einer zweiten Position bewegt werden kann, wobei die Klammern freigegeben werden, um die Einsatzkonfiguration anzunehmen, wobei die Hülse so ausgelegt ist, dass sie sich durch Drehen in Bezug auf die Kammern zwischen den ersten und zweiten Positionen bewegt.

2. Gerät nach Anspruch 1, wobei das Formgedächtnis wenigstens teilweise thermisch aktiviert wird.

3. Gerät nach Anspruch 1 oder 2, wobei die Hülse eine Mehrzahl sich axial erstreckender, länglicher Öffnungen (22) aufweist, die als Eintrittsstellen für Klammern dienen, die in die Kammern geladen werden sollen, und wobei die Hülse eine Mehrzahl von länglichen Austrittsrillen (23) zum Entladen der Klammern in die Einsatzkonfiguration aufweist.

4. Gerät nach Anspruch 3, wobei die Austrittsrillen von den Öffnungen radial versetzt sind.

5. Gerät nach einem der Ansprüche 1 bis 4, ferner umfassend einen Träger (20), der die Kammern umfasst, und wobei die Kammern so ausgelegt sind, dass sie sich anfänglich zum Laden der Klammern in den Träger mit den Öffnungen ausrichten, und anschließend, wenn die Hülse zum Entladen der Klammern in Bezug auf den Träger gedreht wird, mit den Austrittsrillen ausrichten.

6. Gerät nach einem der Ansprüche 1 bis 5, ferner umfassend Mittel zum Verhindern von vorzeitiger Entladung der Klammern.

7. Gerät nach einem der Ansprüche 1 bis 5, wobei jede Kammer (27) eine Arretierung (26) definiert, in welche sich die zugehörige Klammer verformen kann.

8. Gerät nach einem der Ansprüche 1 bis 7, ferner umfassend einen Hebel, einen Antriebsmechanismus (4) und einen Handgriff (2) mit einem Abzug (3), wobei der Hebel es ermöglicht, dass der Antriebsmechanismus (4) nach anfänglicher Aktivierung des Abzugs (3) gedreht wird.

9. Gerät nach Anspruch 1 in der Form eines medizinischen Klammerapparats.

10. Gerät nach Anspruch 9, umfassend eine Kappe (30) zur Aufnahme eines Transplantats (40) an einem Ende des Klammergeräts, bis die Klammern eingesetzt werden.

## Revendications

1. Dispositif comprenant :
plusieurs chambres (25) destinées à recevoir plusieurs agrafes associées (50), possédant chacune une mémoire de forme qui permet à l'agrafe d'adopter une configuration redressée, lorsqu'elle est placée dans une agrafeuse, et une configuration déployée pour effectuer une suture lorsqu'elle est libérée de l'agrafeuse,
**caractérisé en ce que** le dispositif comprend en outre :
une chemise (5) mobile par rapport aux chambres entre une première position, dans laquelle les agrafes sont emprisonnées par la chemise dans les chambres dans la configuration redressée, et une deuxième position, dans laquelle les agrafes sont libérées pour adopter la configuration déployée, dans laquelle la chemise est conçue pour se déplacer entre la première et la deuxième positions en effectuant une rotation par rapport aux chambres.

2. Dispositif selon la revendication 1, dans lequel la mémoire de forme est au moins partiellement activée de manière thermique.

3. Dispositif selon la revendication 1 ou la revendication 2, dans lequel la chemise possède plusieurs ouvertures allongées se prolongeant de manière axiale (22) qui servent de points d'entrée pour les agrafes à charger dans les chambres et dans lequel la chemise possède plusieurs rainures de sortie allongées (23) pour libérer les agrafes dans la configuration déployée.

4. Dispositif selon la revendication 3, dans lequel les rainures de sortie sont décalées de manière radiale par rapport aux ouvertures.

5. Dispositif selon l'une quelconque des revendications 1 à 4, comprenant en outre un support (20) comprenant les chambres et les chambres sont disposées pour s'aligner initialement avec les ouvertures pour charger les agrafes dans le support et s'aligner ensuite avec les rainures de sortie lorsque la chemise est mise en rotation par rapport au support pour libérer les agrafes.

6. Dispositif selon l'une quelconque des revendications 1 à 5, comprenant en outre un moyen pour restreindre la libération prématurée des agrafes.

7. Dispositif selon l'une quelconque des revendications 1 à 5, dans lequel chaque chambre (27) définit une détente (26) dans laquelle l'agrafe associée est libre de se déformer.

8. Dispositif selon l'une quelconque des revendications 1 à 7, comprenant en outre une tige, un mécanisme d'entraînement (4) et une poignée (2) munie d'une gâchette (3), dans lequel la tige permet la mise en rotation du mécanisme d'entraînement (4) lorsque la gâchette (3) a été activée initialement.

9. Dispositif selon la revendication 1, sous la forme d'une agrafeuse médicale.

10. Dispositif selon la revendication 9 comprenant un capuchon (30) pour maintenir un greffon (40) à une extrémité de l'agrafeuse jusqu'à ce que les agrafes soient déployées.
